# EUROPEAN PATENT SPECIFICATION

(11) **EP 0 393 708 B1**
(45) Date of publication and mention of the grant of the patent: **29.06.1994**
(21) Application number: 90107555.6
(22) Date of filing: 20.04.1990
(51) Int. Cl.: C12P 13/10, C12N 1/20

(54) **Process for producing L-ornithine by fermentation**
Verfahren zur Herstellung von L-Ornithin durch Fermentation
Procédé de production de L-Ornithine par fermentation

(30) Priority: 20.04.1989 JP 101011/89
(43) Date of publication of application: 24.10.1990
(73) Proprietor: AJINOMOTO CO., INC., Tokyo 104 (JP)
(72) Inventor: Tsuchida, Takayasu, c/o Kawasaki Plant, Kawasaki-ku, Kawasaki-shi, Kanagawa-ken (JP); Uchibori, Haruo, c/o Kawasaki Plant, Kawasaki-ku, Kawasaki-shi, Kanagawa-ken (JP); Nishimoto, Yoshitaka, c/o Kawasaki Plant, Kawasaki-ku, Kawasaki-shi, Kanagawa-ken (JP)
(74) Representative: Strehl Schübel-Hopf Groening & Partner

(56) References cited:
- GB-A- 1 140 827
- GB-A- 1 178 005
- CHEM. PHARM. BULLERIN, vol. 17, 1969; KAWAMURA et al., pp. 1902-1909#
- PATENT ABSTRACTS OF JAPAN, unexamined applications, C field, vol. 7, no. 7, 12 January 1983, The Patent Office Japanese Govt.; p. 166 C 144#
- PATENT ABSTRACTS OF JAPAN, unexamined applications, C field vol. 10, no. 310, 22 October 1986, The Patent Office Japanese Govt.; p. 37 C 379#

## Description

L-Ornithine is an important component of drugs for stimulating liver functions, as a component of total amino acid preparations and other drugs. The present invention relates to an improved process for producing L-ornithine by fermentation.

As microorganisms belonging to the genus Brevibacterium and the genus Corynebacterium which have L-ornithine productivity, there are known citrulline or arginine auxotrophs belonging to the genus Brevibacterium (Japanese Patent Publication No. 43-8712), citrulline or arginine auxotrophs belonging to the genus Bacillus (Japanese Patent Publication No. 43-10996), citrulline or arginine auxotrophs belonging to the genus Arthrobacter (Japanese Patent Publication No. 44-24303), variants of arginine auxotroph having a resistance belonging to the genus Corynebacterium (Japanese Patent Application Laid-Open No. 53-24096), or variants belonging to the genus Corynebacterium having a resistance to 2-thiazole-alanine, sulfaguanidine or 2-fluoropyruvic acid (Japanese Patent Application Laid-Open No. 61-119194).

The aim of the present invention is to increase the fermentation yield of the process for producing L-ornithine by means of a microorganism strains and thus to allow the industrial production of L-ornithine.

The investigations underlying the present invention were carried out in order to solve the above problem.

As a result of investigations to improve the productivity of microorganisms belonging to the genus Brevibacterium, the genus Corynebacterium and the genus Arthrobacter, which are capable of producing L-ornithine, and to find excellent strains having a further improved fermentation yield, it has been discovered that strains to which a resistance to mycophenolic acid or ornithinol has been imparted are capable of producing L-ornithine in a higher yield than conventional L-ornithine-producing strains.

Subject matter of the present invention is a process for producing L-ornithine which comprises culturing in a liquid medium an L-ornithine producing microorganism belonging to the genus Brevibacterium, the genus Corynebacterium or the genus Arthrobacter having auxotrophy for at least one of the compounds arginine or citrulline and having resistance to at least one compound selected from mycophenolic acid and ornithinol, and collecting L-ornithine from the medium.

The microorganisms used in the present invention are variants belonging to the genus Brevibacterium, the genus Corynebacterium or the genus Arthrobacter, having a resistance to mycophenolic acid or ornithinol and the capability of producing L-ornithine. The resistance may be either mycophenolic acid resistance or ornithinol resistance; or, the microorganisms may also have both resistances. To obtain the variants of the present invention, L-ornithine productivity may first be imparted to wild strains as described below and mycophenolic acid resistance or ornithinol resistance may then be imparted. Alternatively, mycophenolic acid resistance or ornithinol resistance may firstly be imparted and L-ornithine productivity such as arginine auxotrophy or citrulline auxotrophy may then be imparted.

Wild strains which can be parent strains of the variants of the present invention are known bacteria belonging to the genus Brevibacterium, the genus Corynebacterium or the genus Arthrobacter, especially those known as Coryneform L-glutamic acid producing bacteria. Representative examples are the following bacteria.

| | |
|---|---|
| Brevibacterium divaricatum | ATCC 14020 |
| Brevibacterium flavum | ATCC 14067 |
| Brevibacterium lactofermentum | ATCC 13869 |
| Brevibacterium saccharolyticum | ATCC 14066 |
| Corynebacterium acetoacidophilum | ATCC 13870 |
| Corynebacterium glutamicum | ATCC 13032 |
| Arthrobacter citreus | ATCC 17775 |

To induce the variants of the present invention from these parent strains by mutation, a conventional method for mutation which comprises contacting these strains with N-methyl-N'-nitro-N-nitrosoguanidiine, or other conventional mutagens can be appropriately used.

A specific method for mutation of the variant of the present invention and the relationship between a concentration of mycophenolic acid or ornithinol and growth of the strain are shown below.

### Method for inducing mutation

Bacterial cells of Arthrobacter citreus AJ 12441 (FERM BP-2341) having arginine auxotrophy, which had been grown in bouillon agar slants at 30°C for 24 hours, were suspended in M/30 phosphate buffer solution. To the cell suspension was added 100 »g/ml of N-methyl-N'-nitro-N-nitrosoguanidine. The mixture was kept at 30°C for 30 minutes to react. The cells were then collected. After thoroughly washing with M/30 phosphate buffer solution, the cells were inoculated on a medium having the following composition and cultured at 31.5°C for 5 days. Among the bacterial strains grown on the medium, large colonies were mainly picked up. Most of the picked up strains had a high L-ornithine productivity.

| Composition of Medium (pH 7.0) | |
|---|---|
| Component | Content |
| Glucose | 1 g/dl |
| Urea | 0.2 g/dl |
| KH₂PO₄ | 0.1 g/dl |
| MgSO₄.7H₂O | 0.1 g/dl |
| FeSO₄.7H₂O | 0.002 g/dl |
| MnSO₄.7H₂O | 0.002 g/dl |
| Biotin | 100 »g/l |
| Thiamine hydrochloride | 100 »g/l |
| L-Arginine | 15 mg/dl |
| Mycophenolic acid | 0.1 g/dl |
| Agar | 2.0 g/dl |
| pH | 7.2 |

From the strains grown on the agar medium, Arthrobacter citreus AJ 12442 (FERM BP-2342; arginine auxotrophy and citrulline auxotrophy and mycophenolic acid resistance) was obtained as having high productivity of L-ornithine.

Ornithinol-resistant Brevibacterium lactofermentum AJ 12444 (FERM BP-2344) was bred from Brevibacterium lactofermentum AJ 12443 (FERM BP-2343; arginine and citrulline auxotrophy) in a manner similar to the method described above except that L-arginine and mycophenolic acid in the medium described above were changed to chemicals required by them or imparting resistance to them; Corynebacterium glutamicum AJ 12445 (FERM BP-2345; arginine and citrulline auxotrophy and vitamin P resistance, mycophenolic acid resistance and ornithinol resistance) was collected from Corynebacterium glutamicum AJ 11589 (FERM BP-5644; arginine and citrulline auxotrophy and vitamin P resistance) by the method of breeding twice.

The mycophenolic acid or ornithinol resistance of the thus obtained variants was compared with that of the parent strains.

Onto a medium composed of 0.5 g/dl of glucose, 0.15 g/dl of urea, 0.15 g/dl of ammonium sulfate, 0.3 g/dl of KH₂PO₄, 0.1 g/dl of K₂HPO₄, 0.01 g/dl of MgSO₄.7H₂O, 0.1 mg/dl of CaCl₂.2H₂O, 100 »g/l of biotin, 100 »g/l of thiamine hydrochloride, 0.002 g/dl of FeSO₄.7H₂O, 0.002 g/dl of MnSO₄.7H₂O, 15 mg/dl of L-arginine and mycophenolic acid or ornithinol in the amounts shown in the table and adjusted to pH 7.0, there was inoculated a suspension of the cells in sterile water, which cells had been obtained by culturing in natural medium (1 g/dl of polypeptone, 1 g/dl of yeast extract and 0.5 g/dl of NaCl, pH 7.0) slant at 31.5°C for 24 hours. After shake culture in a test tube for 24 hours, the growth degree was determined in terms of turbidity.

**Table 1**

| Strain | Concentration of Mycophenolic Acid (%) | | | | | Concentration of Ornithinol (%) | | | | |
|---|---|---|---|---|---|---|---|---|---|---|
| | 0 | 0.05 | 0.1 | 0.2 | 0.3 | 0 | 0.05 | 0.1 | 0.2 | 0.3 |
| Arthrobacter citreus AJ 12441 (FERM BP-2341) | 0.95 | 0.45 | 0 | 0 | 0 | | | | | |
| Arthrobacter citreus AJ 12442 (FERM BP-2342) | 0.96 | 0.90 | 0.90 | 0.85 | 0.70 | | | | | |
| Brevibacterium lactofermentum AJ 12443 (FERM BP-2343) | | | | | | 0.95 | 0.90 | 0.50 | 0.10 | 0 |
| Brevibacterium lactofermentum AJ 12444 (FERM BP-2344) | | | | | | 0.95 | 0.95 | 0.90 | 0.85 | 0.85 |
| Corynebacterium glutamicum AJ 11589 (FERM P-5644) | 0.09 | 0.40 | 0.10 | 0 | 0 | 0.92 | 0.90 | 0.65 | 0.20 | 0 |
| Corynebacterium glutamicum AJ 12445 (FERM BP-2345) | 0.95 | 0.90 | 0.85 | 0.90 | 0.80 | 0.95 | 0.90 | 0.95 | 0.80 | 0.85 |

Other microorganisms belonging to the genus Brevibacterium, the genus Corynebacterium or the genus Arthrobacter to which the process of the present invention is applicable may also be collected as described above.

Media used for culturing such variants may be conventional media containing carbon sources, nitrogen sources, inorganic ions, substances satisfying the auxotrophy described above and, if necessary, other organic trace nutrients including vitamins etc. As carbon sources, there are preferably used carbohydrates such as glucose, sucrose, etc., organic acids such as acetic acid, etc. As nitrogen sources, there are preferably used ammonia water, ammonia gas, ammonium salts, etc. As inorganic ions, potassium ions, sodium ions, magnesium ions, phosphate ions and the like are appropriately added to media, depending upon necessity.

Incubation is carried out under aerobic conditions. When the incubation is carried out while adjusting pH of the medium to a range from 4 to 8 at a temperature of from 25°C to 37°C, more preferable results can be obtained. Thus, during culturing for 1 to 7 days, remarkable amounts of L-ornithine are produced and accumulated in the medium.

For collecting L-ornithine from the culture solution, conventional method such as a method using ion exchange resin, etc. can be used.

By the process of the present invention, the yield of L-orinithine produced by fermentation can be improved and the production costs can be reduced.

The present invention is described with reference to the example below.

### Example

A medium containing 10 g/dl of glucose, 7 g/dl of (NH₄)₂SO₄, 0.15 g/dl of KH₂PO₄, 0.04 g/dl of MgSO₄.7H₂O, 1 mg/dl of FeSO₄.7H₂O, 1 mg/dl of MnSO₄.4H₂O, 100 »g/l of thiamine hydrochloride, 100 »g/l of biotin, 60 mg of soybean protein acid hydrolysate (calculated as total nitrogen), 15 mg/dl of L-arginine and 5 g/dl of calcium carbonate (independently sterilized) was adjusted to pH 7.2 and 25 ml of the medium was charged in a flask with a shoulder having a 500 ml volume followed by sterilization with heating. One platinum loop of the strain shown in Table 2 was inoculated in the medium and shaken for 4 days while keeping at 31.5°C. L-Ornithine was produced and accumulated in the culture solution of each strain in the amount shown in Table 2.

**Table 2**

| Strain | Property | Amount of L-Ornithine Accumulated (g/dl) |
|---|---|---|
| Arthrobacter citreus AJ 12441 (FERM BP-2341) | Arg⁻ x Cit⁻ | 3.5 |
| Arthrobacter citreus AJ 12442 (FERM BP-2342) | Arg⁻ x Cit⁻ x MPA^{γ} | 5.0 |
| Brevibacterium lactofermentum AJ 12443 (FERM BP-2343) | Arg⁻ x Cit⁻ | 4.7 |
| Brevibacterium lactofermentum AJ 12444 (FERM BP-2344) | Arg⁻ x Cit⁻ x ORL^{γ} | 5.5 |
| Corynebacterium glutamicum AJ 11589 (FERM P-5644) | Arg⁻ x Cit⁻ x VP^{γ} | 4.6 |
| Corynebacterium glutamicum AJ 12445 (FERM BP-2345) | Arg⁻ x Cit⁻ x VP^{γ} x MPA^{γ} x ORL^{γ} | 5.3 |
| Arg⁻ : arginine auxotrophy Cit⁻ : citrulline auxotrophy MPA^{γ} : mycophenolic acid resistance ORL^{γ} : ornithinol resistance VP^{γ} : vitamin P resistance | | |

## Claims

1. A process for producing L-ornithine which comprises culturing in a liquid medium an L-ornithine producing microorganism belonging to the genus Brevibacterium, the genus Corynebacterium or the genus Arthrobacter having auxotrophy for at least one of the compounds arginine or citrulline and having resistance to at least one compound selected from mycophenolic acid and ornithinol, and collecting L-ornithine from the medium.

2. A process according to claim 1, wherein the microorganism is selected from
Arthrobacter citreus AJ 12442 (FERM BP-2342)
Brevibacterium lactofermentum AJ 12444 (FERM BP-2344)
Corynebacterium glutamicum AJ 12445 (FERM BP-2345).

3. Microorganism, selected from
Arthrobacter citreus AJ 12442 (FERM BP-2342)
Brevibacterium lactofermentum AJ 12444 (FERM BP-2344)
Corynebacterium glutamicum AJ 12445 (FERM BP-2345).

## Patentansprüche

1. Verfahren zur Herstellung von L-Ornithin, das darin besteht, daß in einem flüssigen Medium ein L-Ornithin produzierender Mikroorganismus des Genus Brevibacterium, des Genus Corynebacterium oder des Genus Arthrobacter gezüchtet wird, der Auxothrophie für mindestens eine der Verbindungen Arginin oder Citrullin hat und der resistent gegenüber mindestens einer Verbindung, ausgewählt unter Mycophenolsäure und Ornithinol, ist, und daß das L-Ornithin aus dem Medium gewonnen wird.

2. Verfahren nach Anspruch 1, bei dem der Mikroorganismus unter
Arthrobacter citreus AJ 12442 (FERM BP-2342)
Brevibacterium lactofermentum AJ 12444 (FERM BP-2344)
Corynebacterium glutamicum AJ 12445 (FERM BP-2345)
ausgewählt wird.

3. Mikroorganismus, der ausgewählt ist unter
Arthrobacter citreus AJ 12442 (FERM BP-2342)
Brevibacterium lactofermentum AJ 12444 (FERM BP-2344)
Corynebacterium glutamicum AJ 12445 (FERM BP-2345)

## Revendications

1. Procédé de production de L-ornithine, lequel comprend la culture dans un milieu liquide d'un micro-organisme producteur de L-ornithine appartenant au genre Brevibacterium, au genre Corynebacterium ou au genre Arthrobacter, présentant une auxotrophie pour au moins un des composés arginine ou citrulline et une résistance à au moins un composé sélectionné parmi l'acide mycophénolique et l'ornithinol, et le recueil de la L-ornithine dans le milieu.

2. Procédé selon la revendication 1, dans lequel le micro-organisme est sélectionné parmi
Arthrobacter citreus AJ 12442 (FERM BP-2342)
Brevibacterium lactofermentum AJ 12444 (FERM BP-2344)
Corynebacterium glutamicum AJ 12445 (FERM BP-2345).

3. Micro-organisme sélectionné parmi
Arthrobacter citreus AJ 12442 (FERM BP-2342)
Brevibacterium lactofermentum AJ 12444 (FERM BP-2344)
Corynebacterium glutamicum AJ 12445 (FERM BP-2345).
